# EUROPEAN PATENT SPECIFICATION

(11) **EP 0 797 375 B1**
(45) Date of publication and mention of the grant of the patent: **05.06.2002**
(21) Application number: 97103641.3
(22) Date of filing: 05.03.1997
(51) Int. Cl.: H05B 33/14, H05B 33/12, C07C 211/58, G03G 5/06, H01L 33/00

(54) **Organic electroluminescent device with new hole transporting materials**
Organische elektrolumineszente Vorrichtung mit neuen Ladungslöchertransportmaterialien
Dispositif organique électroluminescent contenant de nouveaux matériaux transportant des trous de charge

(30) Priority: 19.03.1996 US 616833
(43) Date of publication of application: 24.09.1997
(73) Proprietor: MOTOROLA, INC., Schaumburg, IL 60196 (US)
(72) Inventor: Shi, Song Q., Phoenix, Arizona 85044 (US); So, Franky, Tempe, Arizona 85284 (US); Lee, Hsing-Chung, Calabasas, California 91302 (US)
(74) Representative: Gibson, Sarah Jane

(56) References cited:
- EP-A- 0 713 148
- EP-A- 0 731 625
- EP-A- 0 774 883

## Description

### Field of the Invention

This invention relates to an organic electroluminescence device and, more specifically, to a device with a new hole transporting material.

### Background of the Invention

Organic electroluminescent (EL) devices are generally composed of three layers of organic molecules sandwiched between transparent and metallic electrodes, the three layers including an electron transporting layer, an emissive layer and a hole transporting layer. Organic EL devices are attractive owing to low driving voltage and a potential application to full color flat emissive displays. Though significant lifetime has been achieved in the prior art (See US Patent No. 4,720,432, entitled "Electroluminescent Device with Organic Luminescent Medium", issued January 19, 1988), further improvement is needed for applications where high brightness is required. In general, the hole transporting materials used in the hole transporting zone of an organic EL device are the most vulnerable parts which are susceptible to thermal degradation by physical aggregation or recrystallization.

To address the thermal degradation problem of the hole transporting materials in an organic EL device, several schemes have been proposed: one being a double-layer hole transporting configuration (see US Patent 5,256,945, entitled "Organic Electroluminescent Element", issued October 26, 1993); one being usage of materials of high glass transition temperatures (US Patent 5,061,569, entitled "Electroluminescent Device with Organic Electroluminescent Medium", issued October 29, 1991).

It is a purpose of this invention to provide an organic EL device with a new hole transporting material, which has high thermal stability.

### Summary of the Invention

An organic EL device comprises a cathode, an electron transporting layer, an emitting layer, a hole transporting layer and an anode which are laminated in sequence, where said hole transporting layer is made of a substance represented by the following chemical formula I: 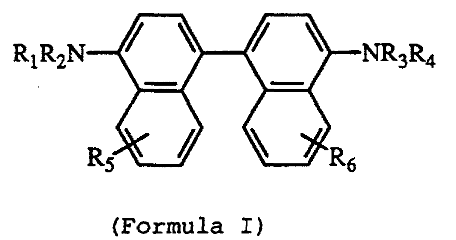 where R₁,R₂,R₃,R₄,R₅,R₆ each independently represent a hydrogen atom, an alkyl group of from 1 to 6 carbon atoms, a halogen group, a cyano group, a nitro group, or an aryl group of from 6 to 15 carbon atoms, a fused aromatic group, an alkoxy group, an alkylamine group, an aryloxy group, or an arylamine group.

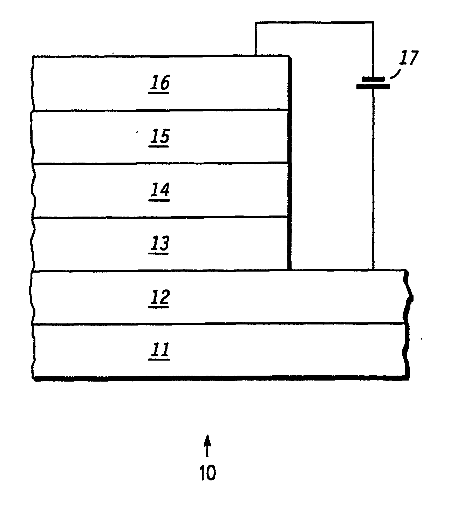

### Brief Description of the Drawing

The single figure is a simplified cross-sectional view of a light emitting diode in accordance with the present invention.

### Description of the Preferred Embodiments

The present invention is directed to an organic light emitting device which, in general, consists of thin layers of organic molecules sandwiched between transparent and metallic electrodes.

The single Figure illustrates in a simplified cross-sectional view, one embodiment of an organic light emitting device (LED) 10. Organic LED 10 includes a transparent substrate 11 which in this specific embodiment is a glass or plastic plate having a relatively planar upper surface. A transparent electrically conductive layer 12 is deposited on the planar surface of substrate 11 so as to form a relatively uniform electrical contact. A hole transporting layer 13 made of organic hole transporting materials is deposited on the surface of conductive layer 12. An emissive organic layer 14 made of a host emissive material containing a guest fluorescent dopant is deposited onto the surface of layer 13. Then an electron transporting layer 15 made of an electron transporting material is deposited on the surface of layer 14 and a second electrically conductive layer 16 is deposited on the upper surface of layer 15 to form a second electrical contact.

In this embodiment, conductive layer 16 is formed of any of a wide range of metals or alloys in which at least one metal has a work function less than 4.0 eV. By the proper selection of material for conductive layer 16, the work functions of the materials making up layers 15 and 16 are substantially matched to reduce the required operating voltage and improve the efficiency of organic LED 10. Additional information on work function matching is disclosed in a copending U.S. Patent Application entitled "Organic LED with Improved Efficiency", filed 12 September 1994, bearing serial number 08/304,454, and assigned to the same assignee.

In this embodiment conductive layer 12 is a p-type contact and conductive layer 16 is an n-type contact. The negative terminal of a potential source 17 is connected to conductive layer 16 and the positive terminal is connected to conductive layer 12. When a potential is applied between layers 12 and 16 by means of potential source 17, electrons injected from the n-type contact (layer 16) are transported through organic layer 15 and into organic layer 14 (the emissive layer) and holes injected from the p-type contact (layer 12) are transported through organic layer 13 and into organic layer 14 (the emissive layer), where upon an electron and a hole recombination a photon is emitted.

It has been recognized in the prior art that the hole transporting materials made up of tertiary aromatic amines are the least thermally stable materials in organic electroluminescent devices. Especially, when an organic EL device is driven at high electric current density, the hole transporting materials in the hole transporting layer tend to aggregate or reorganize to form islands, domains and pinholes. The change in film uniformity and homogeneity of hole transporting materials causes initially the gradual degradation in device luminance and eventually the device failure.

Generally, the glass transition temperature (Tg) of a material is an indicator for thermal stability of the resulting amorphous films. Higher glass transition temperature offers higher thermal stability for an amorphous film. A large molecule weight and strong steric hindrance to stacking are two of the main factors that determine tie glass transition temperature of an organic material.

In an effort to improve the reliability of organic EL devices, Shirota (see J. Phys. Chem. 1993, 97, 6240 and reference within) and Adachi (Appl. Phys. Lett, 1995, 66 (20), 2679) have designed and prepared several symmetric globular tertiary aromatic amines with high Tg, yielding homogeneous amorphous films for organic EL devices applications. In the prior art, the following compounds represented by HMT-1, HMT-2, HMT-3 and HMT-4 are a few organic hole transporting materials with Tg above 75 ºC and give excellent lifetimes when used in organic EL devices.

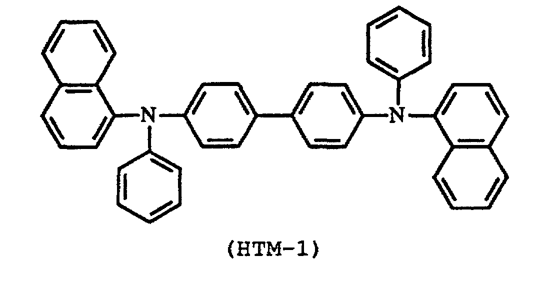

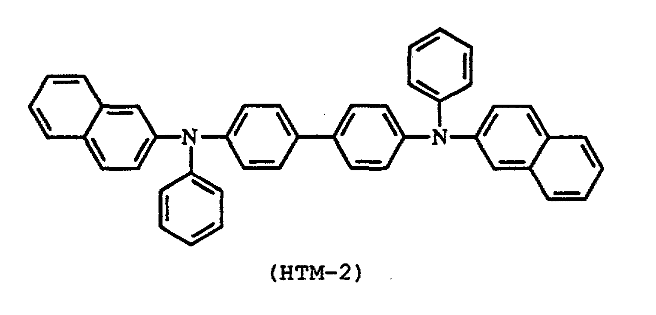

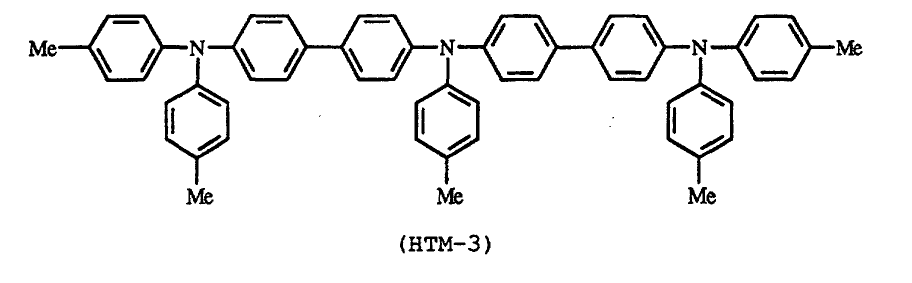

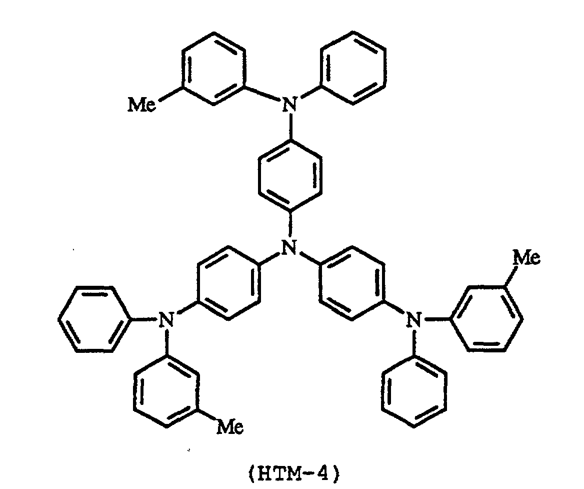

It is a purpose of this invention to provide a new class of aromatic amines having high glass transition temperatures for use in organic light emitting diode.

According to present invention, an organic EL device as illustrated in LED 10 comprises a cathode, an electron transporting layer, an emitting layer, a hole transporting layer and an anode which are laminated in sequence, where said hole transporting layer is made of a substance with central binaphthyl group represented by the following chemical formula: 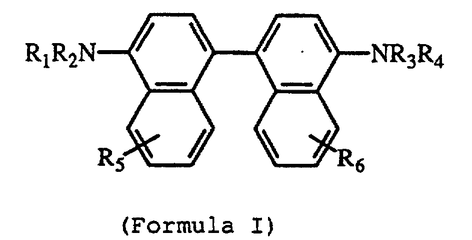 where R₁,R₂,R₃,R₄,R₅,R₆ each independently represent a hydrogen atom, an alkyl group of from 1 to 6 carbon atoms, a halogen group, a cyano group, a nitro group, or an aryl group of from 6 to 15 carbon atoms, a fused aromatic group, an alkoxy group, an alkylamine group, an aryloxy group, or an arylamine group.

In this specific embodiment, it is desirable that the hole transporting material used in the hole transporting layer in organic EL device 10 has glass transition temperatures above 75 ºC. However, it is preferred that hole transporting material having glass transition temperature greater then 90 ºC is to be used to achieve a long lasting organic EL device.

The following is a partial list of examples of aromatic tertiary amines satisfying the requirement of the present invention:

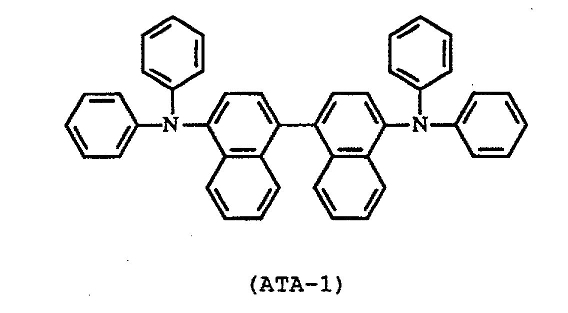

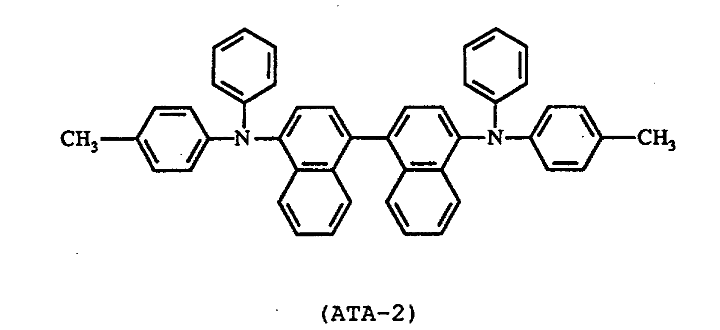

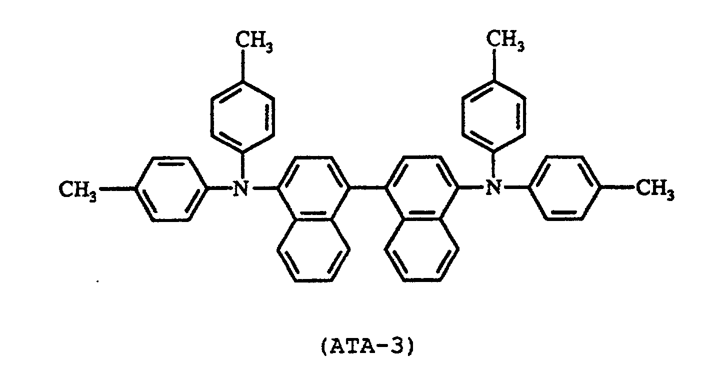

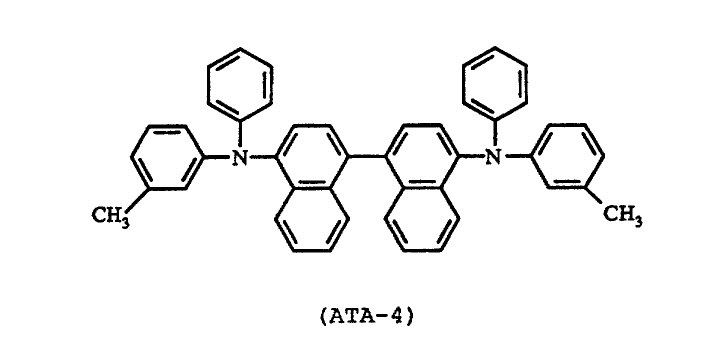

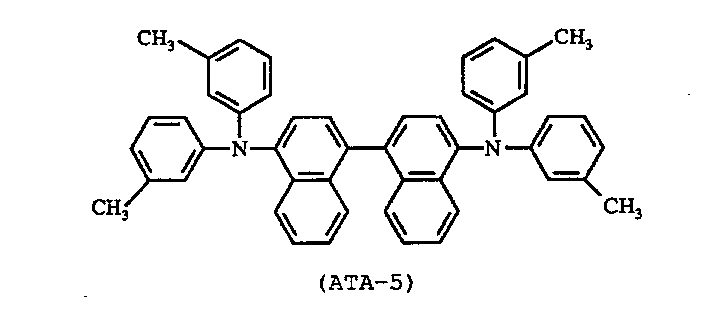

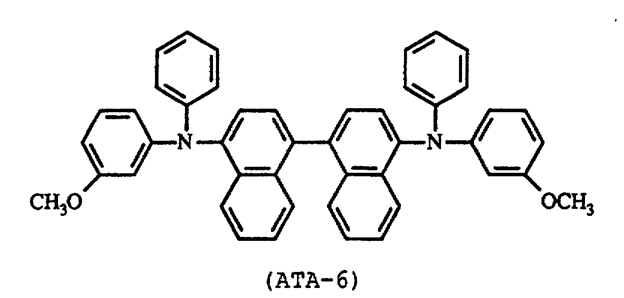

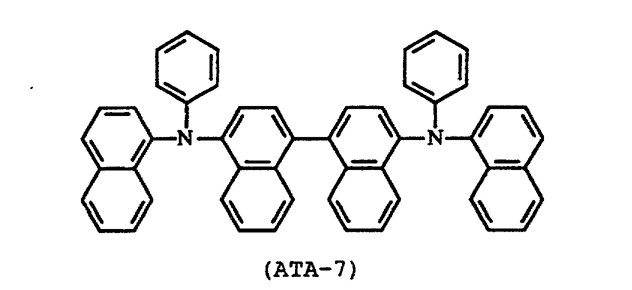

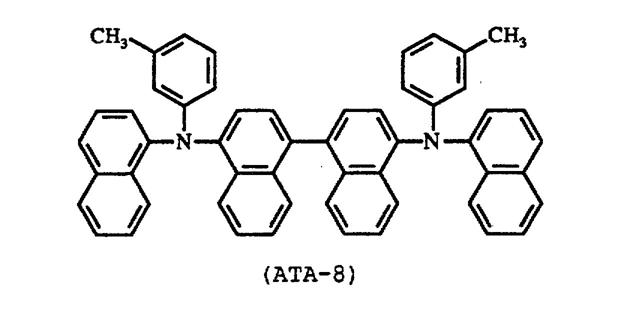

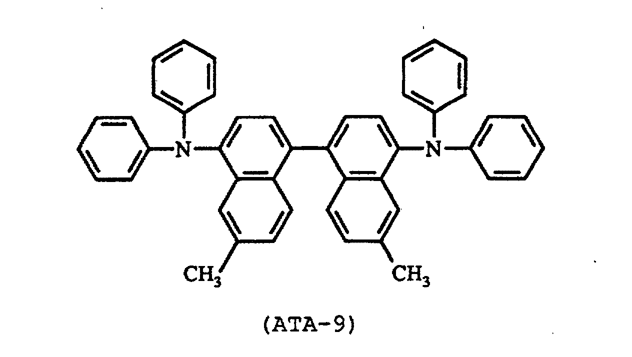

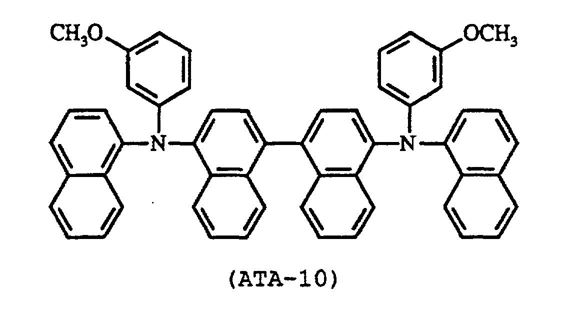

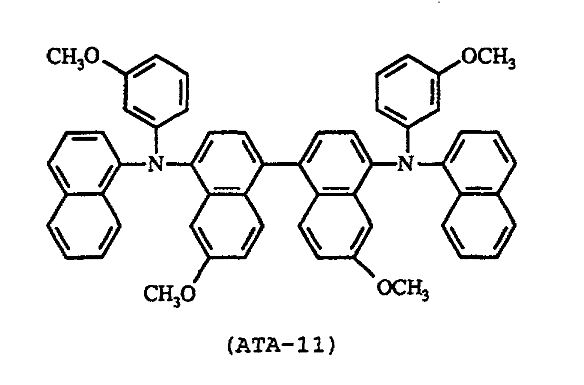

In one alternative embodiment, the hole transporting layer in LED 10 is composed of two layers of hole transporting materials, wherein, at least one layer of the hole transporting material is selected from the substance represent by formula I in accordance with the present invention.

In another alternative embodiment, the hole transporting layer in LED 10 is composed of a layer of mixed hole transporting materials, wherein, at least one of the hole transporting materials is selected from the substance represented by formula I in accordance with present invention.

In this embodiment, the electron transporting layer 15 of LED 10 is preferred to, but not limited to, be selected from the following electron transporting materials:

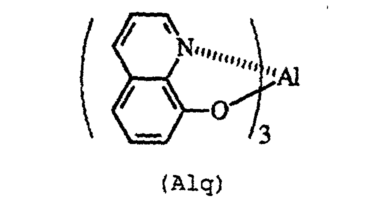

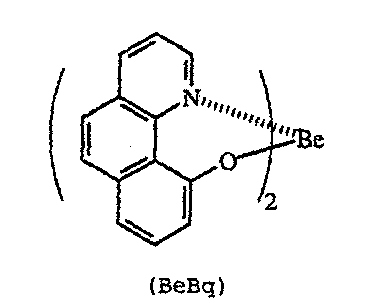

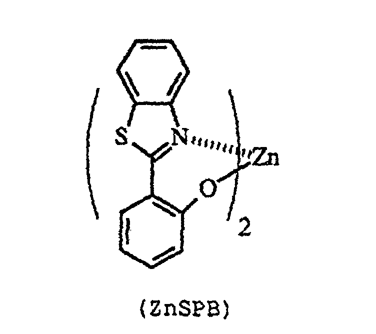

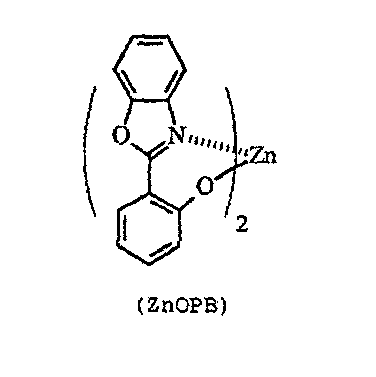

The hole transporting materials used in the embodied organic EL device generally have high glass transition temperatures because of the rigid central binaphthyl group. Since higher glass transition temperature generally renders higher thermal stability for an amorphous film, the organic EL devices according to the present invention provide better reliability under high current density driving condition then normal organic EL devices.

While we have shown and described specific embodiments of the present invention, further modifications and improvements will occur to those skilled in the art. I desire it to be understood, therefore, that this invention is not limited to the particular forms shown and I intend in the appended claims to cover all modifications that do not depart from the scope of this invention.

## Claims

1. An organic EL device comprising a cathode (16), an electron transporting layer (15), an emitting layer (14), a hole transporting layer (13) and an anode (12) laminated in sequence, wherein said hole transporting layer (13) includes a material represented by the following chemical formula I: 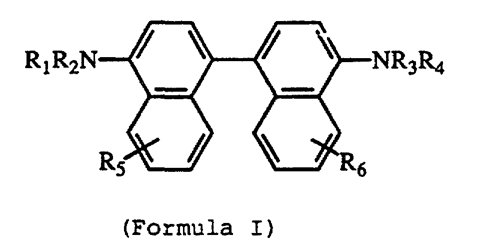 where R₁,R₂,R₃,R₄,R₅,R₆ each independently represent a hydrogen atom, an alkyl group of from 1 to 6 carbon atoms, a halogen group, a cyano group, a nitro group, or an aryl group of from 6 to 15 carbon atoms, a fused aromatic group, an alkoxy group, an alkylamine group, an aryloxy group, or an arylamine group.

2. An organic electroluminescence device as claimed in claim 1, wherein the hole transporting material (13) has a glass transition temperature above 75 ºC.

3. An organic electroluminescence device as claimed in claim 1, wherein the hole transporting material (13) is selected from the following materials:

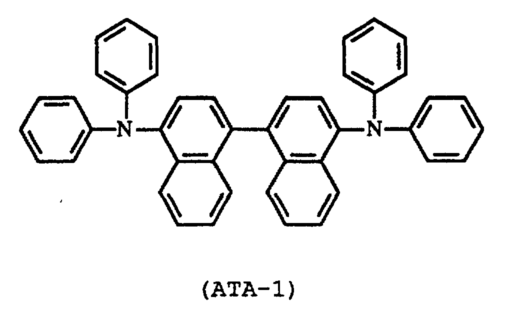

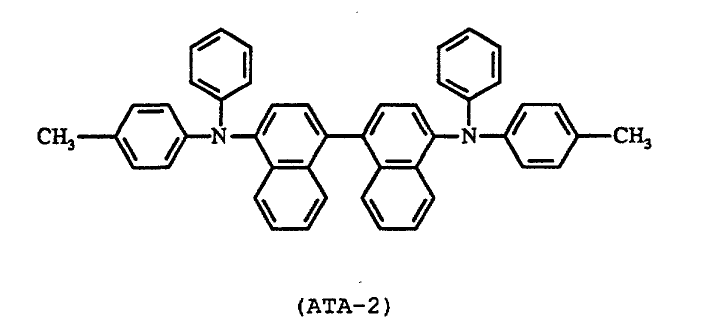

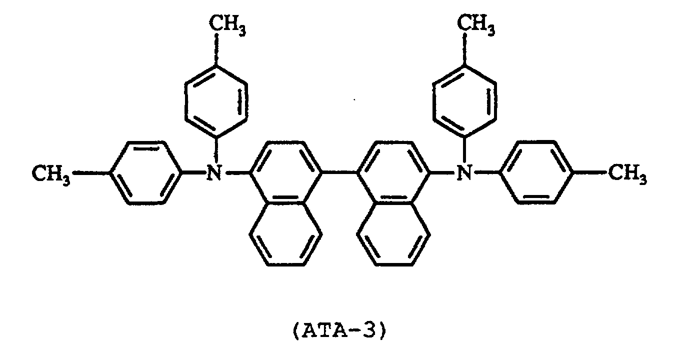

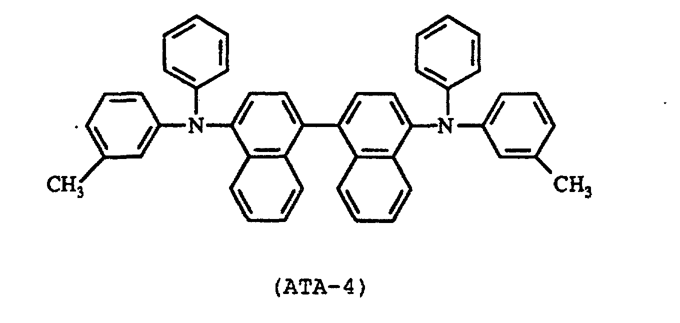

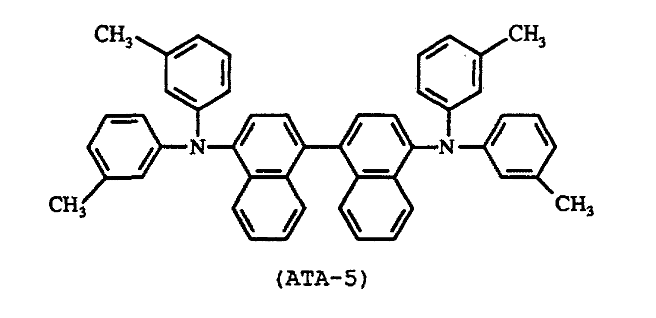

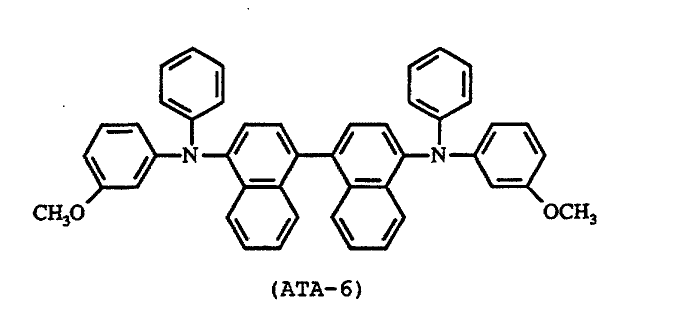

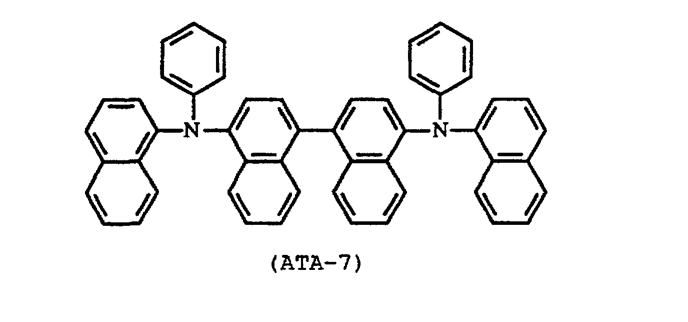

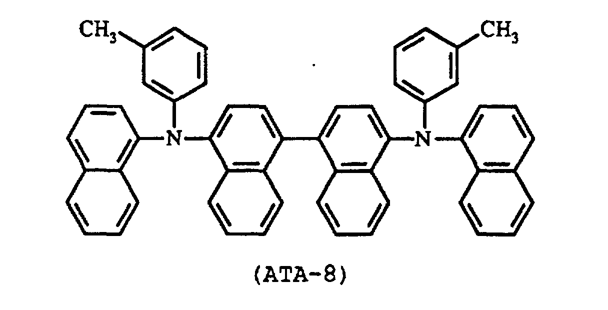

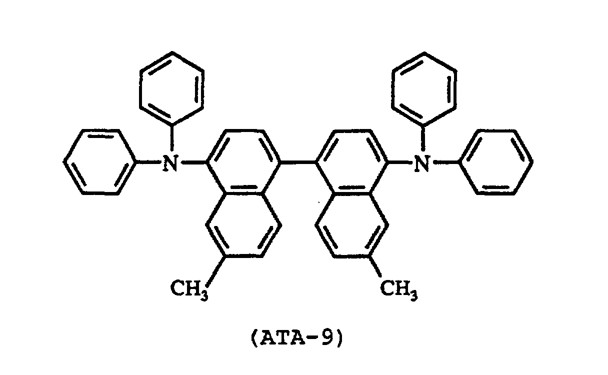

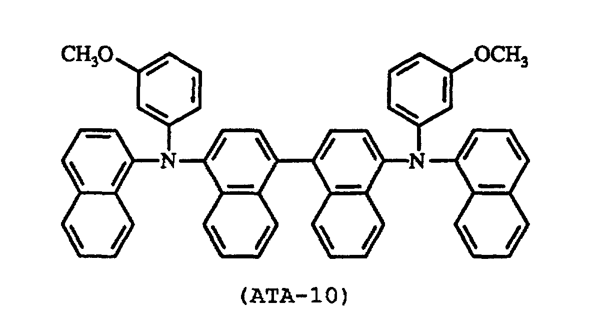

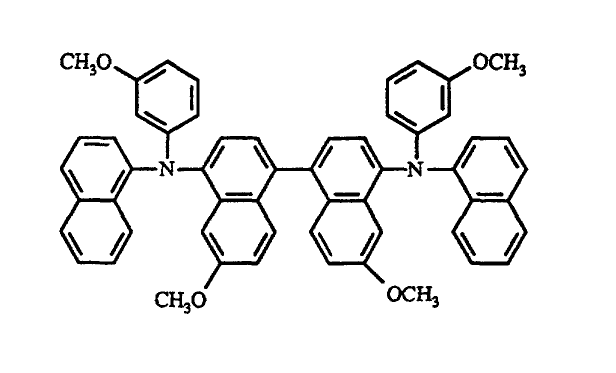

4. An organic electroluminescence device as claimed in claim 1, wherein the electron transporting layer (15) is composed of one of the following materials:

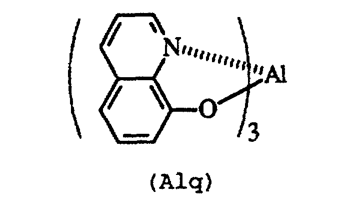

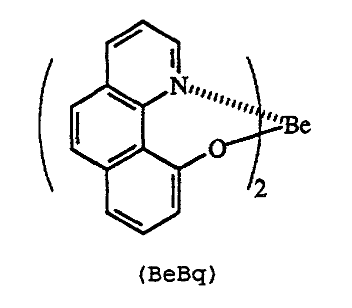

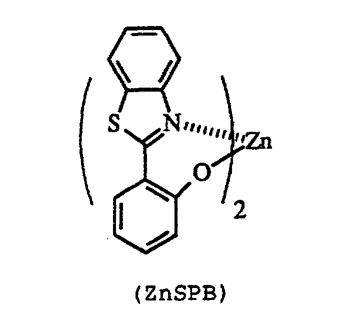

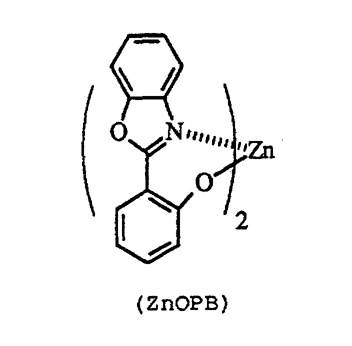

5. An organic electroluminescence device as claimed in claim 1, wherein the hole transporting layer (13) includes two layers of hole transporting materials and at least one layer of the hole transporting material is selected from the substance represent by the following formula I 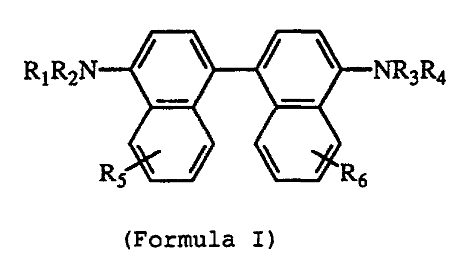 where R₁,R₂,R₃,R₄,R₅,R₆ each independently represent a hydrogen atom, an alkyl group of from 1 to 6 carbon atoms, a halogen group, a cyano group, a nitro group, or an aryl group of from 6 to 15 carbon atoms, a fused aromatic group, an alkoxy group, an alkylamine group, an aryloxy group, or an arylamine group.

6. An organic electroluminescence device as claimed in claim 1, wherein the hole transporting layer (13) includes a layer of mixed hole transporting materials, at least one of the hole transporting materials being selected from a substance represented by the following formula I 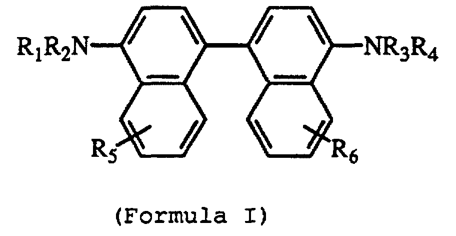 where R₁,R₂,R₃,R₄,R₅,R₆ each independently represent a hydrogen atom, an alkyl group of from 1 to 6 carbon atoms, a halogen group, a cyano group, a nitro group, or an aryl group of from 6 to 15 carbon atoms, a fused aromatic group, an alkoxy group, an alkylamine group, an aryloxy group, or an arylamine group.

## Patentansprüche

1. Organische EL-Vorrichtung, umfassend, der Reihe nach geschichtet, eine Kathode (16), eine elektronentransportierende Schicht (15), eine emittierende Schicht (14), eine löchertransportierende Schicht (13) und eine Anode (12), wobei die löchertransportierende Schicht (13) ein Material enthält das durch die folgende chemische Formel I dargestellt wird: 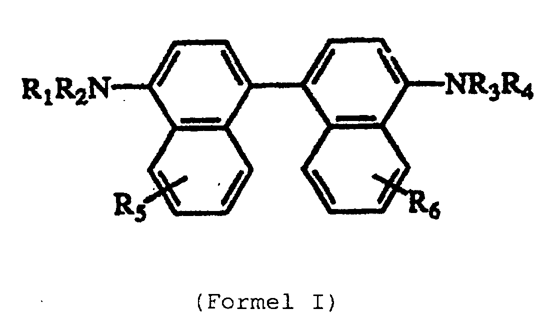 wobei R₁, R₂, R₃, R₄, R₅, R₆ jeweils unabhängig voneinander ein Wasserstoffatom, eine Alkylgruppe mit 1 bis 6 Kohlenstoffatomen, eine Halogengruppe, eine Cyangruppe, eine Nitrogruppe, oder eine Arylgruppe mit von 6 bis 15 Kohlenstoffatomen, eine kondensierte aromatische Gruppe, eine Alkoxygruppe, eine Alkylamingruppe, eine Aryloxygruppe, oder eine Arylamingruppe darstellen.

2. Organische elektrolumineszierende Vorrichtung wie in Anspruch 1 beansprucht, bei der das löchertransportierende Material (13) eine Glas-Übergangstemperatur oberhalb von 75 °C aufweist.

3. Organische elektrolumineszierende Vorrichtung wie in Anspruch 1 beansprucht, bei der das löchertransportierende Material (13) aus den folgenden Materialien ausgewählt ist:

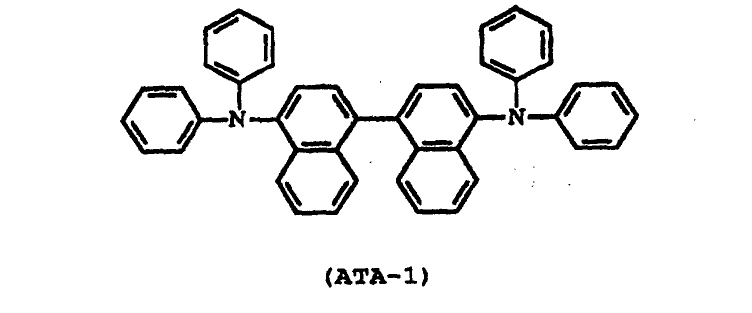

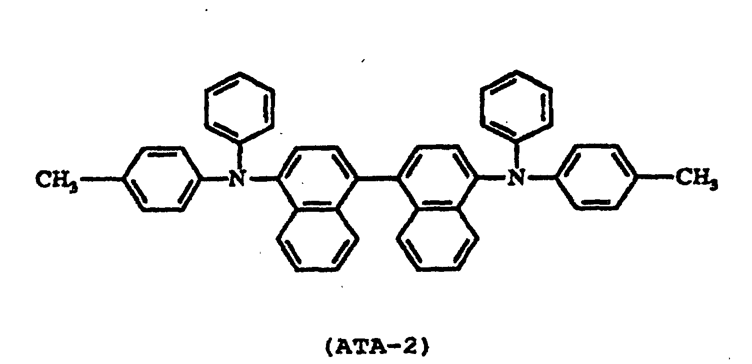

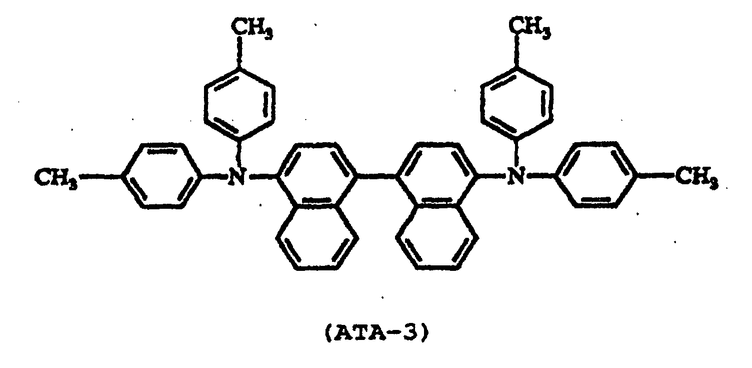

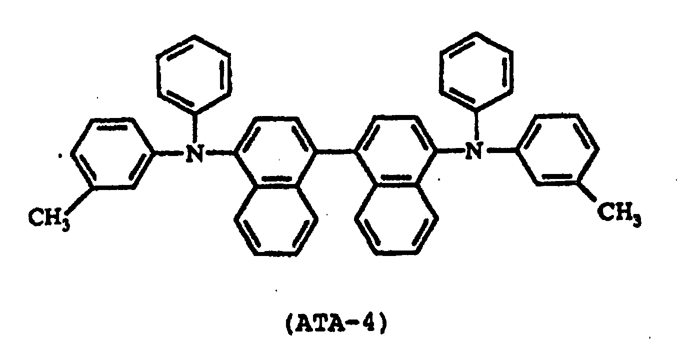

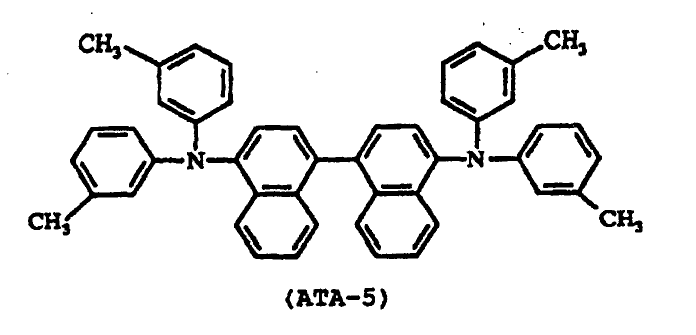

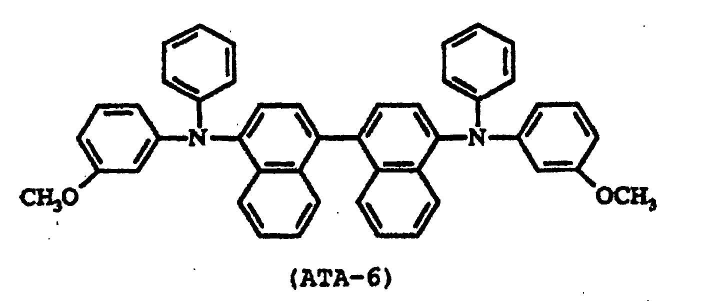

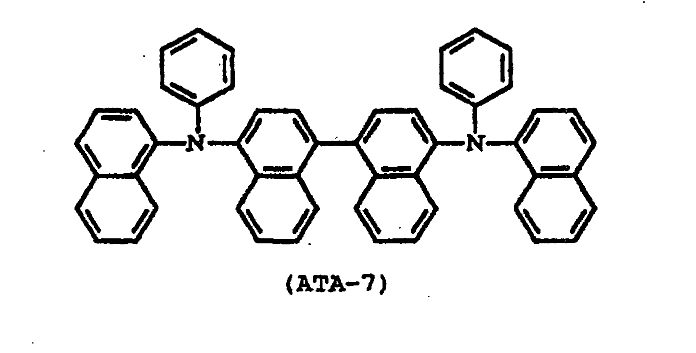

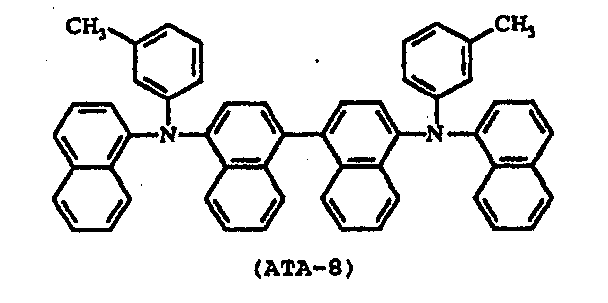

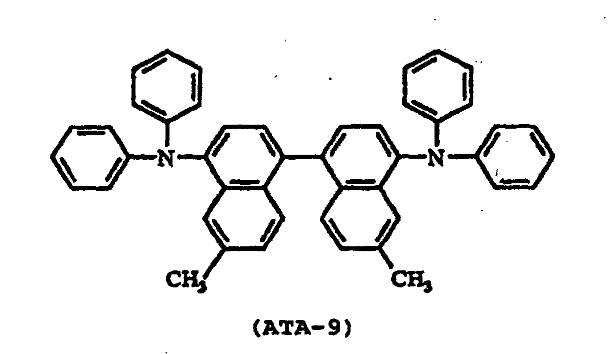

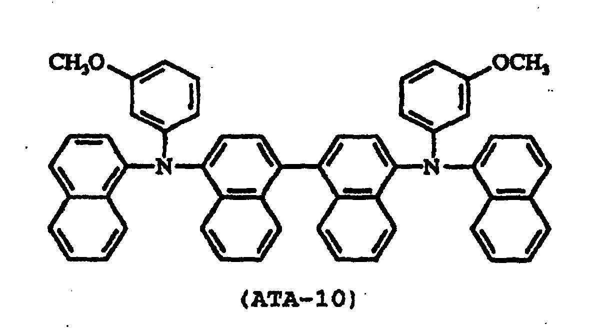

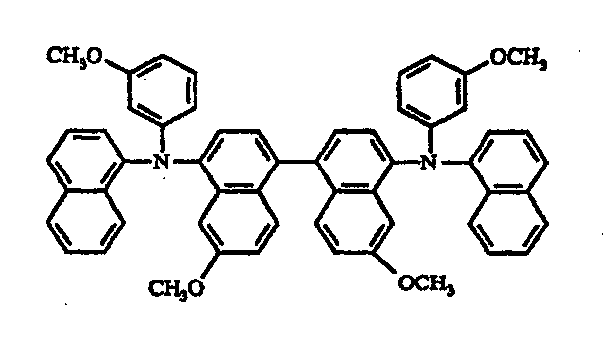

4. Organische elektrolumineszierende Vorrichtung wie in Anspruch 1 beansprucht, bei der die elektronentransportierende Schicht (15) aus einem der folgenden Materialien besteht:

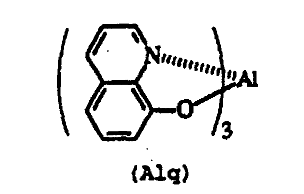

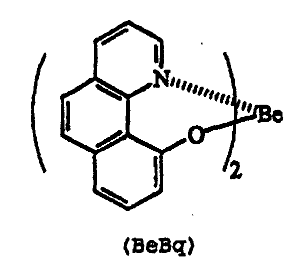

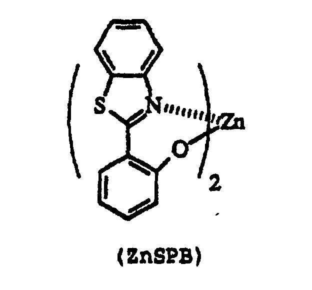

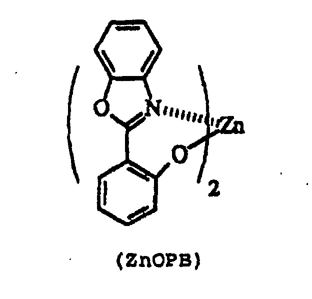

5. Organische elektrolumineszierende Vorrichtung wie in Anspruch 1 beansprucht, bei der die löchertransportierende Schicht (13) zwei Schichten löchertransportierender Materialien enthält, und zumindest eine Schicht des löchertransportierenden Materials aus den von der folgenden Formel I dargestellten Substanzen ausgewählt ist 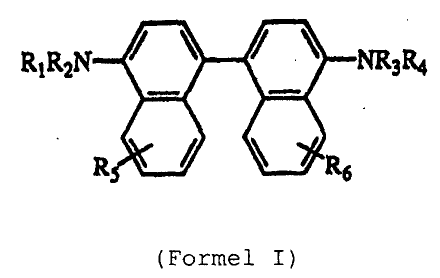 wobei R₁, R₂, R₃, R₄, R₅, R₆ jeweils unabhängig voneinander ein Wasserstoffatom, eine Alkylgruppe mit 1 bis 6 Kohlenstoffatomen, eine Halogengruppe, eine Cyangruppe, eine Nitrogruppe, oder eine Arylgruppe mit von 6 bis 15 Kohlenstoffatomen, eine kondensierte aromatische Gruppe, eine Alkoxygruppe, eine Alkylamingruppe, eine Aryloxygruppe, oder eine Arylamingruppe darstellen.

6. Organische elektrolumineszierende Vorrichtung wie in Anspruch 1 beansprucht, bei der die löchertransportierende Schicht (13) eine Schicht aus gemischten löchertransportierenden Materialien enthält, wobei zumindest eines der löchertransportierenden Materialien aus den von der folgenden Formel I dargestellten Substanzen ausgewählt ist 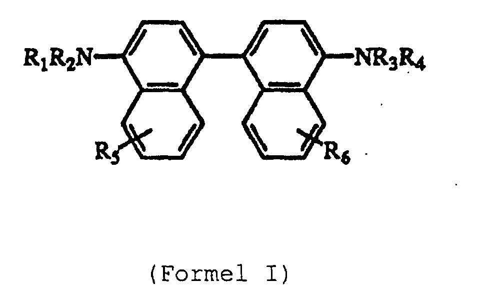 wobei R₁, R₂, R₃, R₄, R₅, R₆ jeweils unabhängig voneinander ein Wasserstoffatom, eine Alkylgruppe mit 1 bis 6 Kohlenstoffatomen, eine Halogengruppe, eine Cyangruppe, eine Nitrogruppe, oder eine Arylgruppe mit von 6 bis 15 Kohlenstoffatomen, eine kondensierte aromatische Gruppe, eine Alkoxygruppe, eine Alkylamingruppe, eine Aryloxygruppe, oder eine Arylamingruppe darstellen.

## Revendications

1. Dispositif électroluminescent organique comprenant une cathode (16), une couche de transport d'électrons (15), une couche émettrice (14), une couche de transport de trous (13) et une anode (12), stratifiées dans cet ordre, dans lequel ladite couche de transport de trous (13) comprend un matériau représenté par la formule chimique I suivante: 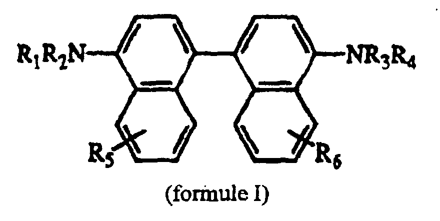 dans laquelle R₁, R₂, R₃, R₄, R₅ et R₆ représentent chacun indépendamment un atome d'hydrogène, un groupe alkyle de 1 à 6 atomes de carbone, un groupe halogéno, un groupe cyano, un groupe nitro, un groupe aryle de 6 à 15 atomes de carbone, un groupe aromatique condensé, un groupe alcoxy, un groupe alkylamino, un groupe aryloxy ou un groupe arylamino.

2. Dispositif électroluminescent organique selon la revendication 1, dans lequel le matériau de transport de trous (13) a une température de transition vitreuse supérieure à 75°C.

3. Dispositif électroluminescent organique selon la revendication 1, dans lequel le matériau de transport de trous (13) est choisi parmi les matériaux suivants:

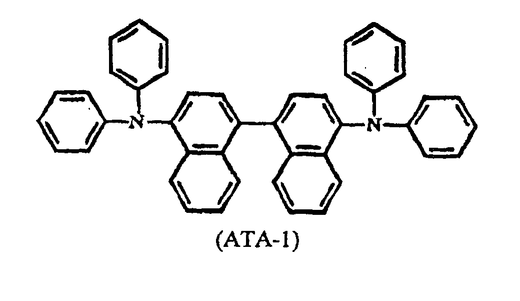

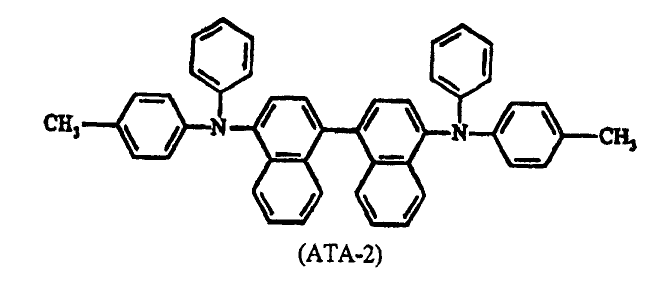

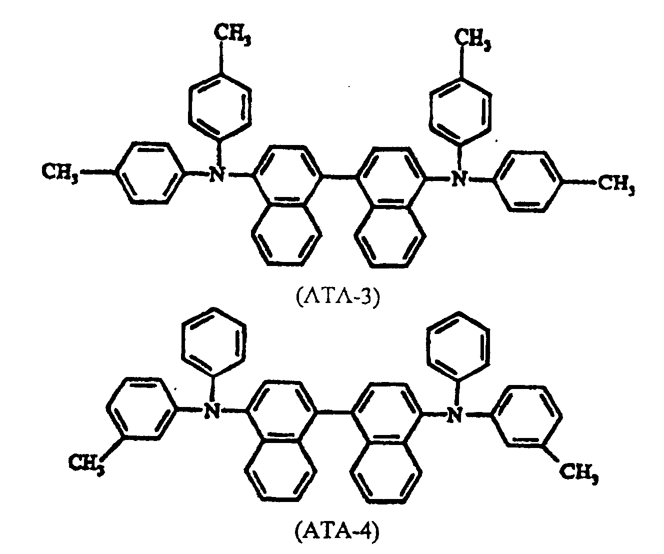

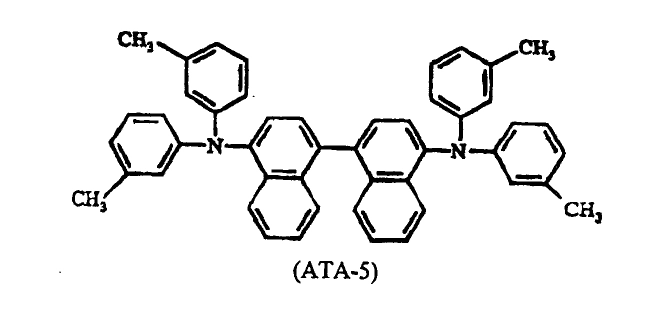

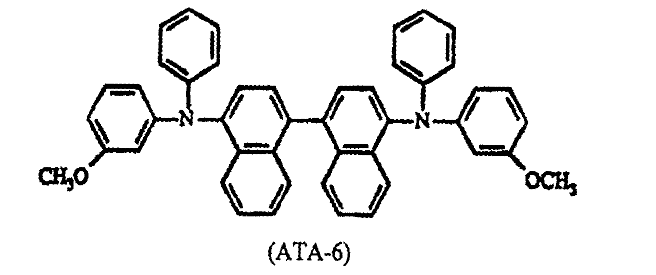

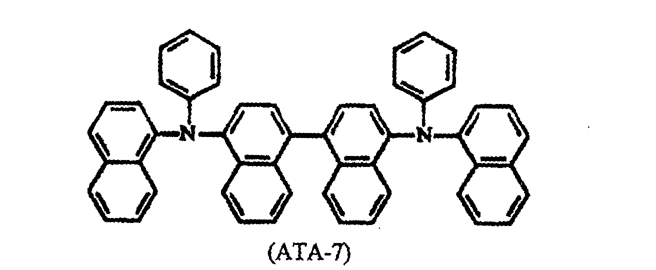

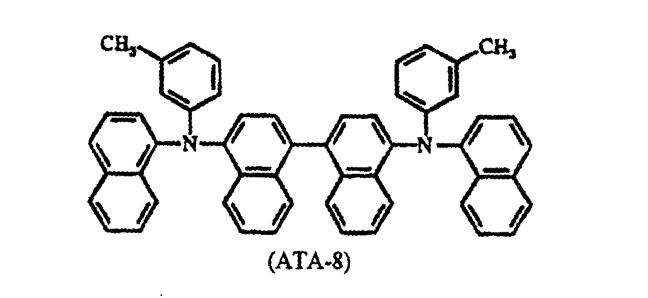

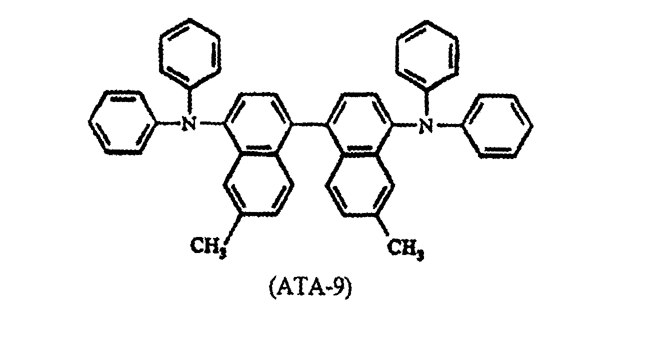

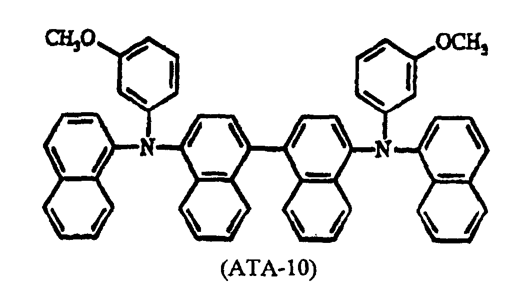

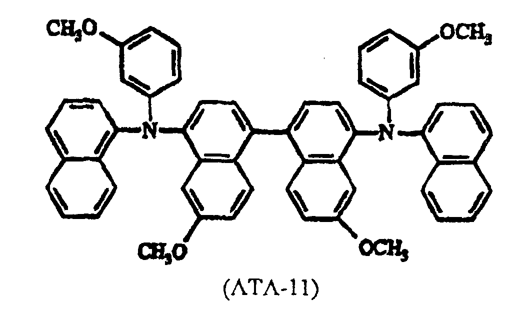

4. Dispositif électroluminescent organique selon la revendication 1, dans lequel la couche de transport d'électrons (15) est composée de l'un des matériaux suivants:

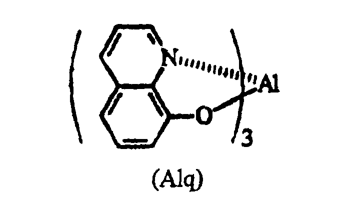

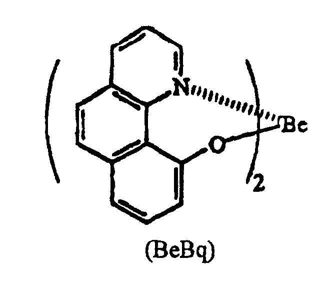

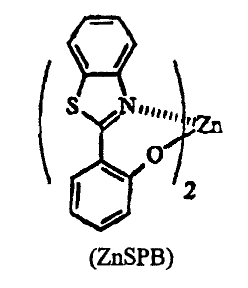

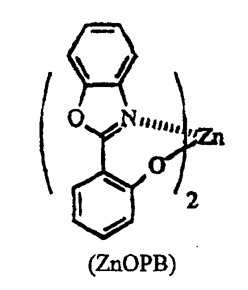

5. Dispositif électroluminescent organique selon la revendication 1, dans lequel la couche de transport de trous (13) comprend deux couches de matériaux de transport de trous et au moins une couche de matériau de transport de trous est choisie parmi les substances représentées par la formule I suivante: 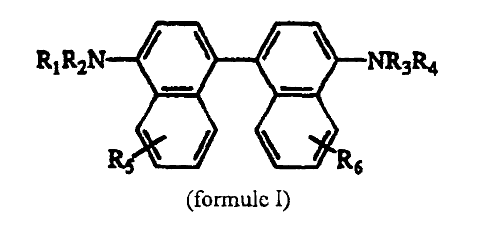 dans laquelle R₁, R₂, R₃, R₄, R₅ et R₆ représentent chacun indépendamment un atome d'hydrogène, un groupe alkyle de 1 à 6 atomes de carbone, un groupe halogéno, un groupe cyano, un groupe nitro, un groupe aryle de 6 à 15 atomes de carbone, un groupe aromatique condensé, un groupe alcoxy, un groupe alkylamino, un groupe aryloxy ou un groupe arylamino.

6. Dispositif électroluminescent organique selon la revendication 1, dans lequci la couche de transport de trous (13) comprend une couche de matériaux de transport de trous mélangés, au moins un des matériaux de transport de trous étant choisi parmi les substances représentées par la formule I suivante : 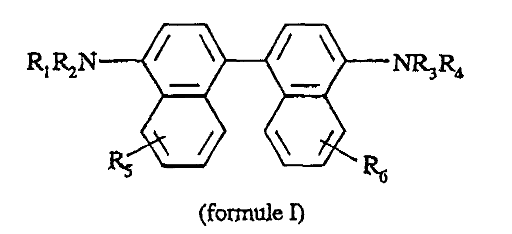 dans laquelle R₁, R₂, R₃, R₄, R₅ et R₆ représentent chacun indépendamment un atome d'hydrogène, un groupe alkyle de 1 à 6 atomes de carbone, un groupe halogéno, un groupe cyano, un groupe nitro, un groupe aryle de 6 à 15 atomes de carbone, un groupe aromatique condensé, un groupe alcoxy, un groupe alkylamino, un groupe aryloxy ou un groupe arylamino.
